(19) Europäisches Patentamt

European Patent Office

Office européen des brevets

(11) EP 4 501 245 A1

(12) **EUROPEAN PATENT APPLICATION**

(43) Date of publication:
05.02.2025 Bulletin 2025/06

(21) Application number: 23194483.6

(22) Date of filing: 31.08.2023

(51) International Patent Classification (IPC):
*A61B 8/08* (2006.01)     *A61B 8/00* (2006.01)

(52) Cooperative Patent Classification (CPC):
A61B 8/5223; A61B 8/085; A61B 8/463;
A61B 8/467

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 01.08.2023 US 202363530122 P

(71) Applicant: Koninklijke Philips N.V.
5656 AG Eindhoven (NL)

(72) Inventors:
• KRUECKER, Jochen
  Eindhoven (NL)
• BALASUNDAR, Iyyavu Raju
  Eindhoven (NL)
• CHEN, Alvin
  5656AG Eindhoven (NL)

(74) Representative: Philips Intellectual Property &
Standards
High Tech Campus 52
5656 AG Eindhoven (NL)

(54) **ULTRASOUND IMAGING CLASSIFICATION WITH SIZE-BASED METRICS**

(57) In accordance with the present disclosure, the systems and methods enable a physician or other healthcare professional to quickly isolate, review, and confirm the most relevant findings / detections. As a result, the systems and methods described herein can consider the size of the findings in a way that can be tailored to the patient's background and clinical condition. According to particular aspects of the present disclosure, a user interface is described that enables a physician-user an efficient way of determining whether the multitude of detections in the cineloop are clinically relevant for the current patient and clinical context, which improves the feasibility and usefulness of point-of-care ultrasound systems for lung ultrasound imaging. A computer-implemented method (400) for automated, size-based analysis of ultrasound cineloops using a point-of-care ultrasound system having an electronic device that is local (i.e., in proximity to) a subject undergoing ultrasound imaging is illustrated according to certain aspects of the present disclosure.

```
400

410 → ACQUIRE ULTRASOUND CINELOOP

420 → SELECT ONE OR MORE SIZE THRESHOLDS

430 → FOR EACH CINELOOP FRAME, DETERMINE
        LOCALIZATIONS, SIZES, AND CONFIDENCE
        SCORES FOR FEATURES OF INTEREST

440 → CREATE SIZE METRICS AND CONFIDENCE
        METRICS FOR DETECTED FEATURES OF INTEREST

450 → CLASSIFY ACQUIRED CINELOOP BASED ON SIZE
        METRICS AND CONFIDENCE METRICS

460 → DISPLAY CLASSIFICATION RESULTS AND
        LOCALIZATIONS OF DETECTED FEATURES
```

FIG. 4

EP 4 501 245 A1

**Description**

FIELD OF THE INVENTION

The present disclosure relates generally to ultrasound imaging systems and methods of performing ultrasound imaging analyses, and more specifically to point-of-care ultrasound systems adapted for performing point-of-care analyses using size-based metrics.

BACKGROUND OF THE INVENTION

Advancements in ultrasound imaging technologies have enabled greater access to ultrasound diagnostic methodologies. Combined with the challenges of moving patients for imaging, point-of-care ultrasound imaging (i.e., ultrasound imaging performed at a point-of-care such as a patient's hospital room, a General Practitioners office, or even at home) has been gaining increasing acceptance for a variety of applications.

SUMMARY OF THE INVENTION

The present disclosure is directed to point-of-care ultrasound systems configured to assist in the real-time medical imaging analysis of a subject and methods of automated, size-based analysis of ultrasound cineloops (i.e., a sequence of ultrasound images)using a point-of-care ultrasound system. The systems and methods of the present disclosure may find particular application in connection with lung ultrasound imaging, especially point-of-care lung ultrasound imaging. While point-of-care ultrasound imaging analysis is becoming more popular, there still exist a number of technical challenges unique to such applications. For example, the identification of clinically-relevant ultrasound features and artifacts requires expertise and can be time consuming. As such, there is a need to provide systems and methods that enable quick and efficient analyses of ultrasound images. Additionally, conventional image processing approaches may also return a number of irrelevant or less important results, which negatively impacts the usefulness of a point-of-care system. Accordingly, the systems and methods provided herein enable automated, size-based analyses of ultrasound cineloops.

The invention is defined by the independent claims. The dependent claims define advantageous embodiments.

According to an embodiment of the present disclosure, a point-of-care ultrasound system configured to assist in the real-time medical imaging analysis of a subject is provided. The system includes an electronic device comprising: a user input device configured to receive user input from a user; a computer-readable storage medium having stored thereon machine-readable instructions to be executed by one or more processors; and one or more processors configured by the machine-readable instructions stored on the computer-readable storage medium to perform the following operations: (i) receive ultrasound imaging data in real-time or substantially real-time, wherein the ultrasound imaging data includes an ultrasound cineloop comprising a plurality of ultrasound imaging frames; (ii) receive user input via the user input device, wherein the user input includes a user selection of one or more size thresholds; (iii) analyze the ultrasound cineloop in real-time or substantially real-time using one or more trained artificial intelligence models to identify one or more features-of-interest; (iv) analyze each of the one or more features-of-interest identified to determine a set of size metrics associated with a corresponding feature-of-interest; and (v) determine a classification output for the ultrasound cineloop based on the user selection of one or more size thresholds and the sets of size metrics associated with the one or more features-of-interest.

In an aspect, the point-of-care ultrasound system may further include an ultrasound probe configured to be coupled to the electronic device, wherein the one or more processors can be configured by the machine-readable instructions stored on the computer-readable storage medium to receive ultrasound imaging data from the ultrasound probe when coupled to the electronic device.

In an aspect, the electronic device may further comprises a display, and the one or more processors can be configured by the machine-readable instructions stored on the computer-readable storage medium to: (vi) display, on the display of the electronic device, a user interface comprising (A) one or more ultrasound imaging frames of the ultrasound cineloop, (B) a set of annotations indicating the one or more features-of-interest identified, and (C) the classification output for the ultrasound cineloop, wherein the classification output includes a feature classification determined based on the user selection of one or more size thresholds and the sets of size metrics associated with the one or more features-of-interest.

In an aspect, the set of annotations may include: (A) one or more bounding boxes identifying a region within at least one ultrasound imaging frame of the ultrasound cineloop, wherein the region contains at least one of the one or more features-of-interest; and (B) a feature type label associated with at least one of the one or more features-of-interest identified.

In an aspect, the user input received via the user input device may include a user selection of at least one size threshold corresponding to at least a first type of feature-of-interest.

In an aspect, the display is a touch-enabled display, the user input device includes the touch-enabled display, and the user input includes touch data received via the touch-enabled display.

According to another embodiment of the present disclosure, a (non-transitory) computer-readable storage medium (e.g., a memory) having stored thereon machine-readable instructions is provided. When executed by one or more processors, the machine-readable instructions cause the one or more processors to perform operations comprising: (i) receive ultrasound imaging data in real-time or substantially real-time, wherein the ultrasound imaging data includes an ultrasound cineloop comprising a plurality of ultrasound imaging frames; (ii) receive user input via a user input device, wherein the user input includes a user selection of one or more size thresholds; (iii) analyze the ultrasound cineloop in real-time or substantially real-time using one or more trained artificial intelligence models to identify one or more features-of-interest; (iv) analyze each of the one or more features-of-interest identified to determine a set of size metrics associated with a corresponding feature-of-interest; and (v) determine a classification output for the ultrasound cineloop based on the user selection of one or more size thresholds and the sets of size metrics associated with the one or more features-of-interest.

In an aspect, the (non-transitory) computer-readable storage medium further includes machine-readable instructions stored thereon that, when executed by the one or more processors, cause the one or more processors to perform operations comprising: (vi) generate a user interface comprising (A) one or more ultrasound imaging frames of the ultrasound cineloop, (B) a set of annotations indicating the one or more features-of-interest identified, and (C) the classification output for the ultrasound cineloop, wherein the classification output includes a feature classification determined based on the user selection of one or more size thresholds and the sets of size metrics associated with the one or more features-of-interest; and (vii) display, on a display associated with the non-transitory computer-readable storage medium, the user interface.

In an aspect, the set of annotations may include: (A) one or more bounding boxes identifying a region within at least one ultrasound imaging frame of the ultrasound cineloop, wherein the region contains at least one of the one or more features-of-interest; and (B) a feature type label associated with at least one of the one or more features-of-interest identified.

In an aspect, the user input received via the user input device may include a user selection of at least one size threshold corresponding to at least a first type of feature-of-interest.

According to still another embodiment of the present disclosure, a computer-implemented method for automated, size-based analysis of ultrasound cineloops using a point-of-care ultrasound system having an electronic device that is local to a subject undergoing ultrasound imaging is provided. The method may include: acquiring, via an ultrasound probe coupled to the electronic device, ultrasound imaging data of the subject in real-time or substantially real-time, wherein the ultrasound imaging data includes an ultrasound cineloop comprising a plurality of ultrasound imaging frames; receiving, via a user input device, a user input comprising a user selection of one or more size thresholds; analyzing the ultrasound cineloop in real-time or substantially real-time using one or more trained artificial intelligence models to identify one or more features-of-interest; analyzing each of the one or more features-of-interest identified to determine a set of size metrics associated with a corresponding feature-of-interest; determining a classification output for the ultrasound cineloop based on the user selection of one or more size thresholds and the sets of size metrics associated with the one or more features-of-interest; and outputting, via the electronic device, the classification output to a user associated with the point-of-care ultrasound system.

In an aspect, the method may further include: generating, via the electronic device, a user input interface comprising an interactive menu for receiving the user selection of one or more size thresholds, wherein the one or more size thresholds includes at least a feature height threshold, a feature width threshold, and/or a feature area threshold; and displaying, via a display of the electronic device, the first user interface; wherein the user input device comprises the user input interface.

In an aspect, the method may further include: generating, via the electronic device, a graphical user interface comprising (A) one or more ultrasound imaging frames of the ultrasound cineloop, (B) a set of annotations indicating the one or more features-of-interest identified, and (C) the classification output for the ultrasound cineloop, wherein the classification output includes a feature classification determined based on the user selection of one or more size thresholds and the sets of sizes metrics associated with the one or more features-of-interest; and displaying, via a display of the electronic device, the graphical user interface; wherein the classification output is outputted to the user associated with the point-of-care ultrasound system as part of the graphical user interface displayed on the display of the electronic device.

In an aspect, the set of annotations can be automatically generated based on the one or more features-of-interest identified by the one or more artificial intelligence models, the set of annotations comprising (A) one or more bounding boxes identifying a region within at least one ultrasound imaging frame of the ultrasound cineloop, wherein the region contains at least one of the one or more features-of-interest; and (B) a feature type label associated with at least one of the one or more features-of-interest identified.

In an aspect, one or more features-of-interest identified by analyzing the ultrasound cineloop using one or more trained artificial intelligence models may be excluded from the classification output based on the user selection of one or more size thresholds and the sets of size metrics associated with the excluded features-of-interest.

These and other aspects of the various embodiments will be apparent from and elucidated with reference to the embodiments described hereinafter.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.

Fig. 1 is a diagram of a point-of-care ultrasound system configured to assist in the real-time medical imaging analysis that is shown in accordance with aspects of the present disclosure.

Fig. 2A is an illustration of a user device and user interface shown in accordance with aspects of the present disclosure.

Fig. 2B is another illustration of a user device and user interface shown in accordance with aspects of the present disclosure.

Fig. 3 is a schematic diagram illustrating the components of a point-of-care ultrasound system in accordance with aspects of the present disclosure.

Fig. 4 is a flowchart illustrating a computer-implemented method for automated, size-based analysis of ultrasound cineloops using a point-of-care ultrasound system in accordance with aspects of the present disclosure.

Fig. 5 is an illustration of the feature analysis of an exemplary ultrasound cineloop that is shown in accordance with aspects of the present disclosure.

Fig. 6 is an illustration of the feature analysis of an exemplary ultrasound cineloop that is shown in accordance with further aspects of the present disclosure.

Fig. 7A is an illustration of the feature classification of an exemplary ultrasound cineloop that is shown in accordance with aspects of the present disclosure.

Fig. 7B is an illustration of the feature classification of an exemplary ultrasound cineloop that is shown in accordance with further aspects of the present disclosure.

DETAILED DESCRIPTION OF EMBODIMENTS

Lung ultrasound (LUS) is an imaging technique used to aid in the evaluation of pulmonary and infectious diseases, such as COVID-19 pneumonia, among many others. Important clinical features such as B-lines, merged B-lines, pleural line changes, consolidations, and pleural effusions, can be visualized under lung ultrasound imaging. However, accurately identifying these clinical features is a serious challenge that involves reviewing an entire acquired cineloop (typically a sequence of 20 to 200 ultrasound imaging frames taken over a short period of time). This task is further complicated because the technical outcomes of the analysis depend significantly upon the size of the individual findings relative to the patient's background and clinical status. That is to say that the relevance of the findings is often related to the size of the detections and depends on the clinical context (e.g., adult vs. pediatric examination, clinical history, etc.).

At the same time, point-of-care LUS is gaining increasingly desirable because of the potential for rapid detection of a variety of pulmonary conditions. However, because point-of-care LUS is not performed in a controlled environment dedicated to the performance of ultrasound imaging, accurate results can be even more difficult to obtain. Identification of clinically relevant ultrasound features and artifacts requires expertise and can be time consuming, which limits the applicability of point-of-care LUS imaging. For example, review and documentation of a LUS exam because a typical LUS exam includes multiple cineloops (up to 12 or 14 cineloops in a complete, standardized lung exam), where each cineloop has to be played (often multiple times) and all images need to be reviewed in order to appreciate the dynamic changes between the images and thereby identify abnormalities. Furthermore, while the detection mechanism needs to be sensitive to the smallest findings that could be relevant for some patients and conditions, there is no universal threshold above/below which a feature is relevant/irrelevant. Similarly, for features that are at least potentially relevant, the size of the feature can be an important diagnostic indicator, but there are no universal thresholds for size-based severity assessment that are applicable to all clinical contexts.

In order to address these challenges and quickly identify and summarize the findings from the many frames of a cineloop into binary or multi-class classification of the presence/absence and/or severity of clinical conditions, provided herein are systems and methods for the automatic, size-based analysis of ultrasound cineloops using a point-of-care ultrasound imaging system that further takes into account the size of the detections and available clinical contextual factors. In accordance with the present disclosure, the systems and methods enable a physician or other healthcare professional to quickly isolate, review, and confirm the most relevant findings / detections. As a result, the systems and methods described herein can consider the size of the findings in a way that can be tailored to the patient's background and clinical condition. For example, the detection of small, sub-centimeter consolidations in an adult recovering from bacterial pneumonia may not be relevant to the physician, but the same findings in a pediatric patient presenting in the ER with signs of pneumonia should be highlighted. In addition, the systems and methods described herein can automatically process the cineloops obtained in real-time or in substantially real-time such that the results are ready for display immediately after (or within seconds of) acquisition of a cineloop.

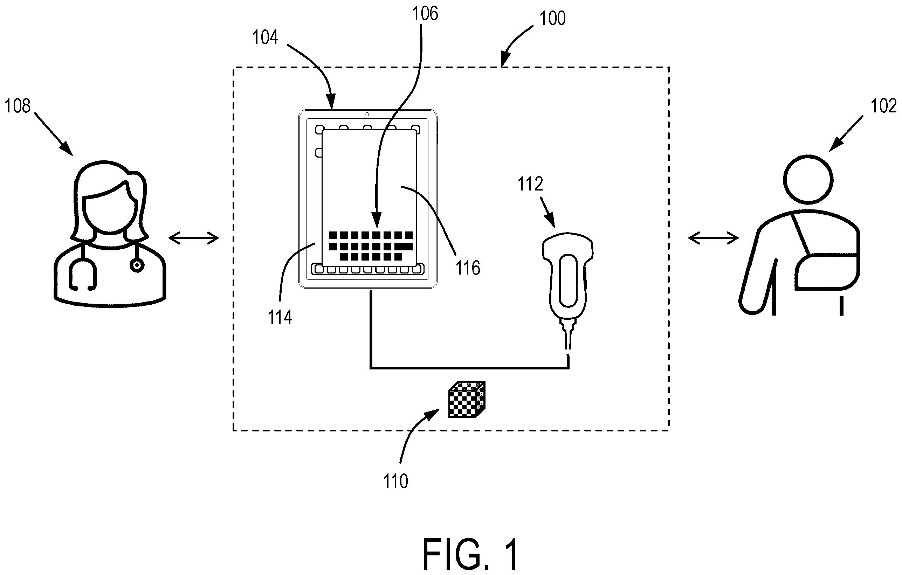

With reference to Fig. 1, a point-of-care ultrasound system 100 configured to assist in the real-time medical imaging analysis of a subject 102 is provided in accordance with various aspects of the present disclosure.

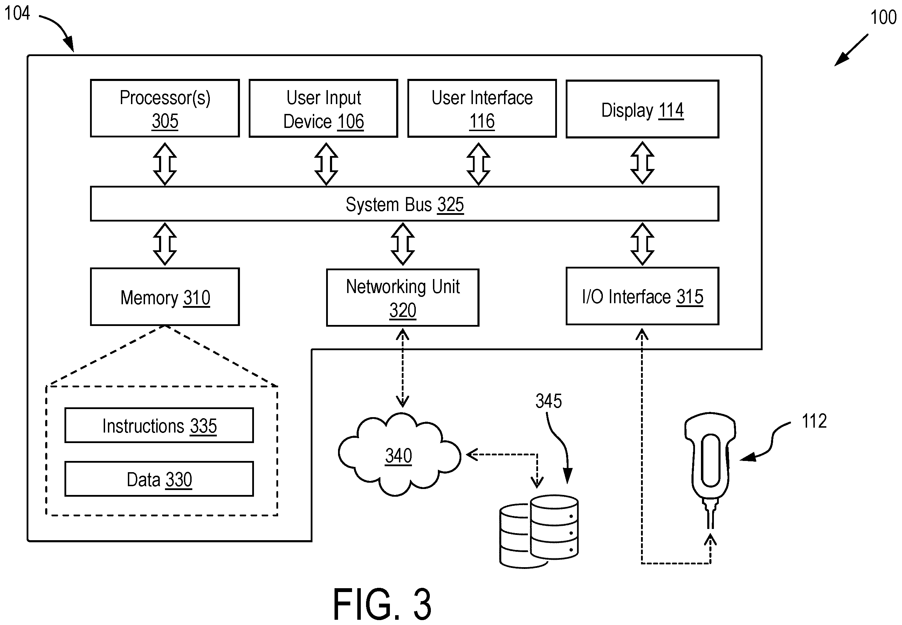

As shown in Fig. 1, the point-of-care ultrasound system 100 may include an electronic device 104 comprising a user input device 106, a computer-readable storage medium (e.g., memory 310 from Fig. 3) having stored thereon machine-readable instructions (e.g., instructions 335 from Fig. 3) to be executed by one or more processors, and one or more processors (e.g., processors 305 from Fig. 3) configured to perform one or more operations of the methods / processes described herein. For example, in particular embodiments, the one or more processors may be configured by machine-readable instructions to perform the following operations: (i) receive ultrasound imaging data 110 in real-time or substantially real-time, wherein the ultrasound imaging data 110 includes an ultrasound cineloop comprising a plurality of ultrasound imaging frames; (ii) receive user input via the user input device 106, wherein the user input includes a user selection of one or more size thresholds; (iii) analyze the ultrasound cineloop in real-time or substantially real-time using one or more trained artificial intelligence models to identify one or more features-of-interest; (iv) analyze each of the one or more features-of-interest identified to determine a set of size metrics associated with each corresponding feature-of-interest; and (v) determine a classification output for the ultrasound cineloop based on the user selection of one or more size thresholds and the sets of size metrics associated with the one or more features-of-interest.

In embodiments, the point-of-care ultrasound system 100 may further include an ultrasound probe 112 configured to be coupled to the electronic device 104 such that the electronic device 104 may receive ultrasound imaging data 110 from the ultrasound imaging probe 112 in real-time or substantially real-time (i.e., while the ultrasound imaging is being performed). In particular embodiments, the ultrasound probe 112 can include an array of transducer elements for transmitting ultrasound waves in a transmission direction (e.g., towards a patient 102) and receiving echo signals in response to the transmitted waves (e.g., from the patient 102). In some embodiments, the ultrasound probe 112 may be reversibly or detachably coupled to the electronic device 104 such that the probe 112 may be connected and disconnected from the electronic device 104. In further embodiments, the ultrasound probe 112 may be portable.

As such, the ultrasound imaging data 110 can include one or more ultrasound cineloop and each cineloop can comprise a plurality of ultrasound imaging frames taken over one or more distinct periods of time. In embodiments, the ultrasound imaging data 110 may be received by the electronic device 104 in a "raw" format and processed by the electronic device 104 into one or more cineloops. Alternatively, the ultrasound imaging data 110 may be preprocessed into one or more cineloops prior to being received by the electronic device 104. That is, the electronic device 104 may receive ultrasound imaging data 110 in an unprocessed or processed format.

In particular embodiments, the electronic device 104 may further include a display 114 configured to display a user interface 116 in accordance with various aspects of the present disclosure. In embodiments, the display 114 may be a touch-enabled display such that the display 114 is also the user input device 106 (e.g., as shown in Fig. 1). In further embodiments, the user input device 106 may include a peripheral device, such as one or more of a keyboard, keypad, trackpad, trackball(s), capacitive keyboard, controller (e.g., a gaming controller), computer mouse, computer stylus / pen, a voice input device, and/or the like.

In embodiments, the user input device 106 can be configured to receive user input from an associated user 108, such as a user 108 performing the ultrasound imaging. In particular embodiments, the user input can include one or more selections (i.e., user selections) of one or more size thresholds. In embodiments, the user input may include a user selection of at least one size threshold corresponding to at least a first type of feature-of-interest. In some embodiments, the user may select at least one size threshold by accepting a default threshold. That is, for example, the user input may include a selection or confirmation of a default setting (e.g., "Consolidations smaller/larger than 1 cm in height or width", or "Pleural Effusions smaller/larger than 0.5 cm in height", etc.).

In embodiments, one or more default settings may be predefined based on one or more clinical contextual parameters associated with the patient 102. Accordingly, in particular embodiments, the user input may include a selection of one or more clinical contextual parameters associated with the patient 102, such as the patient's age, height, weight, sex / gender, stage of physical development, and/or the like. Based on the selection of a clinical contextual parameter or a combination of clinical contextual parameters, the electronic device 104 may be configured to apply one or more predefined (i.e., default) size thresholds (e.g., a first size threshold corresponding to an adult patient, a second size threshold corresponding to a pediatric patient, etc.).

As described in more detail below, the processes described herein involve quantification of one or more features-of-interest from the acquired ultrasound cineloops, including but not limited to features such as B-lines, merged B-lines, pleural line changes, consolidations, and pleural effusions. Each of these features-of-interest may have a corresponding size (height, width, depth). Thus, for example, the user selection may include a size threshold for at least one of these sizing parameters for at least one type of feature-of-interest.

More specifically, the electronic device 104 can be configured to analyze one or more ultrasound cineloops in order to identify one or more features-of-interest. For example, in embodiments, an object detection algorithm may be run to determine a localization and a confidence value for occurrences of a plurality of clinically relevant features in each frame of at least one cineloop. The localization can be determined in the form of a bounding box that tightly encloses the feature, thus also providing a size (height, width) for each detection. However, other forms of localizations are possible such as a binary mask indicating the image pixels that are part of the feature, or a polygon or other shape enclosing the feature. For any such localization, a center and a size (e.g. height, width, area) of the detection can be determined.

Further, a confidence value can be determined for each of the features-of-interest identified. The confidence value may be a normalized value in the range [0.. 1], where 0 indicates lowest confidence, and 1 indicates highest confidence that the feature is present at that location.

In embodiments, the detection algorithm can be based on image processing techniques including thresholding, filtering, and texture analysis, or can be based on machine learning, including one or more artificial intelligence models. In particular embodiments, the trained models may be deep neural networks. For example, in specific embodiments, the detection algorithm may be a Yolo-type network such as Yolo3.

The output of the detection step is, for each frame of the cineloop, a list of detections for one or several types of features-of-interest. Each element in the detection list includes at least the confidence and size (and typically also the position) of detection. Thus, for example, for each frame i and feature f, there is a set of detection parameters:

$$\{w, h; c\}_{f,i}$$

where $w$ and $h$ represent the width and height of the feature $f$, and $c$ represents the confidence value of the feature $f$.

Based on the set of detection parameters, a set of size metrics associated with each corresponding feature-of-interest may be determined. In embodiments, each feature-of-interest detected may be analyzed to determine a set of size metrics that includes one or more statistical parameters for the corresponding feature-of-interest. The statistical parameters may be determined based on the set of detection parameters. For example, for each feature-of-interest detected in an ultrasound cineloop, a statistical parameter (e.g., minimum and/or maximum) may be determined for the measurable parameter (e.g., height, width, and/or area) of the feature-of-interest.

In some embodiments, the size and/or confidence metrics can be tailored to represent clinically relevant parameters derived from the detections, and can be customized for different clinical contexts (e.g., emergency room setting vs. hospital floor setting). For example, one or several metrics may be calculated from the detections as described in US provisional patent application 63/437,206, filed on 2023-Jan-05 (attorneys' reference 2022PF00672).

In embodiments, the electronic device 104 may also be configured to determine a classification output for one or more ultrasound cineloops based on the user input and the set of size metrics associated with one or more features-of-interest. That is, in some embodiments, the electronic device 104 may be configured to determine a classification output for one or more ultrasound cineloops based on the user's selection of one or more size thresholds and/or the user's selection of one or more clinical contextual parameters.

In particular embodiments, the classification output may include a binary classification of the presence and/or absence of a clinical condition, such as the presence and/or absence of one or a combination of features-of-interest. Similarly, the classification output may also include a binary classification of the severity of the clinical condition, including the severity of one or a combination of features-of-interest. For example, in some embodiments, the classification output may include a binary classification indicating the presence or absence of one or more of the following: B-lines, merged B-lines, pleural line changes, consolidations, and pleural effusions. The classification output may additionally include a binary classification indicating that one or more features-of-interest are "severe" or "not severe." In embodiments, the binary classifications may be determined by applying a predetermined set of binary definitions for one or more features-of-interest to the set of size metrics determined for the features-of-interest as well as the user input received.

In further embodiments, the classification output may include a multi-class classification of the presence and/or absence of a clinical condition, such as the presence and/or absence of one or a combination of features-of-interest. Similarly, the classification output may also include a multi-class classification of the severity of the clinical condition, including the severity of one or a combination of features-of-interest. In embodiments, the multi-class classifications may be determined by applying a predetermined set of class definitions for one or more features-of-interest to the set of size metrics determined for the features-of-interest as well as the user input received. For example, the following multi-class definition may be applied to a feature-of-interest "F" based on the maximum height (h) of the feature-of-interest ($Max\{h_{f,i}\} = h_{max}$) determined from the relevant cineloop:

TABLE 1. Multi-Class Definition for Feature-of-interest "F" and Size Metric $h_{max}$

| Condition | Classification Output |
|---|---|
| $h_{max} < 1.0$ cm | Small |
| 1.0 cm $< h_{max} < 2.0$ cm | Medium |
| $h_{max} > 2.0$ cm | Large |

Another example of a multi-class definition for the same size metric is shown below in Table 2:

TABLE 2. Multi-Class Definition for Feature-of-Interest "F" and Size Metric $h_{max}$

| Condition | Classification Output |
|---|---|
| $h_{max} < 0.5$ cm | Score = 0 |
| 0.5 cm $< h_{max} \leq 1.0$ cm | Score = 1 |
| 1.0 cm $< h_{max} \leq 2.0$ cm | Score = 2 |
| 2.0 cm $< h_{max} \leq 4.0$ cm | Score = 3 |
| $h_{max} > 4.0$ cm | Score = 4 |

As described herein, the thresholds / conditions required for this operation can be provided or adjusted by the physician-user 108 in order to adapt the sensitivity or specificity of the classification to the clinical context (e.g., the physician can directly enter a threshold value, or choose options such as "adult" vs. "pediatric" which in turn will result in different thresholds being applied). The size categories or size scores may be calculated and displayed on a per-object or per-frame basis, and/or as a single measurement for the entire cineloop. Further, it should be appreciated that other size metrics may be used instead of the maximum height / depth of any of the detections for a given feature type. For example, in some embodiments, the mean, median, and/or Nth-percentile value may be used, including a combination of these values.

According to aspects of the present disclosure, the analysis of the ultrasound cineloops, determination of the size metrics, and the generation of a classification output may all be performed in real-time or substantially real-time relative to the performance of the ultrasound imaging whereby the ultrasound cineloops are obtained (i.e., within seconds to minutes).

In embodiments, the electronic device 104 may further be configured to display on the display 114 of the electronic device 104 a user interface 116 for interacting dynamically with the obtained LUS imaging and analysis thereof. As described herein, the user interface 116 provides the physician-user 108 an efficient way of determining whether the multitude of detections in the cineloop are clinically relevant for the current patient 102 and clinical context. In particular, the user interface 116 allows the physician-user 108 to determine relevance of the findings in a given clinical context by allowing the user 108 to select one or more size thresholds, and only display detections that exceed the size threshold. Alternatively, the user interface 116 allows the physician-user 108 to determine relevance of the findings in a given clinical context by displaying detections above/below the threshold with different colors, line thickness, shading or other methods to allow the user 108 to differentiate the detections more easily. In further embodiments, the user 108 may be allowed to create defined size categories or size scores based on the size metrics of the detections, adjust these thresholds for the categories/scores, and display detections with different colors, line thicknesses, shadings etc. based on the different categories/scores.

Put another way, the electronic device 104 may further be configured to display on the display 114 of the electronic device 104 a user interface 116 comprising: (A) one or more ultrasound imaging frames from an obtained ultrasound cineloop; (B) a set of annotations indicating one or more features-of-interest detected by the system 100; and (C) a classification output for the ultrasound cineloop.

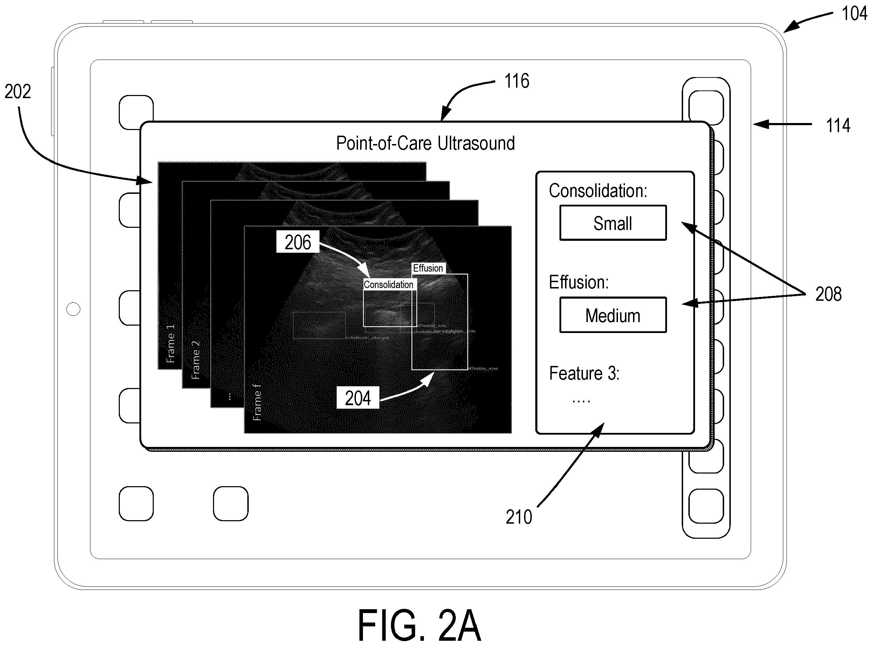

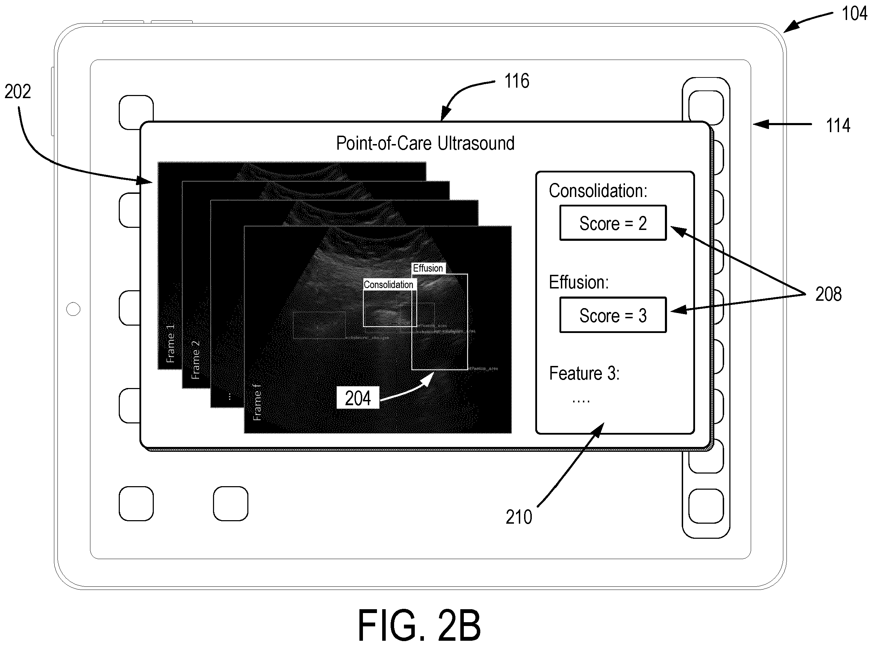

For example, with reference to Fig. 2A and Fig. 2B, the user interface 116 can include one or more ultrasound imaging frames 202 from an obtained ultrasound cineloop, which may be displayed on a display 114 of the electronic device 104. In embodiments, the electronic device 104 may be configured to select the most important or clinically relevant frames from the cineloop for display on the display 114, or may include a sequence of multiple frames to play (i.e., a short clip from the complete cineloop).

As shown in Fig. 2A and Fig. 2B, the user interface 116 can also include a set of annotations 204 that indicate one or more features-of-interest detected by the system 100 in an obtained cineloop. In embodiments, the set of annotations 204 may include one or more bounding polygons (e.g., rectangles, etc.) identifying a region within at least one of the ultrasound frames 202 containing at least one of the features-of-interest identified. In further embodiments, the set of annotations 204 may include one or more feature type labels 206 associated with at least one of the features-of-interest identified. In further embodiments, the user interface 116 can include a classification output 208 determined for at least one of the features-of-interest identified within the ultrasound cineloop. For example, as shown in Fig. 2A and Fig. 2B, the user interface 116 includes an output panel 210 showing the classification output 208 for a "consolidation"-type feature-of-interest and an "effusion"-type feature-of-interest. In the example of Fig. 2A, the classification output 208 includes a qualitative multi-class result for both the consolidation and effusion detections, whereas in the example of Fig. 2B, the classification output 208 includes a score-based multi-class result for both the consolidation and effusion detections.

As described herein, that user interface 116 provides the physician-user 108 a more efficient way of determining whether the multitude of detections in the cineloop are clinically relevant for the current patient 102 and clinical context by allowing the user to visualize the most important frames 202, distinguish different features based on adaptive and customizable annotations, and tailor the classification output to the particular clinical context. Although certain types of annotations are illustrated in Fig. 2A and Fig. 2B, it should also be appreciated that other types of annotations and variations thereof can be utilized, including but not limited to, different colors, line thickness, shading or other methods to allow the user 108 to differentiate the detections more easily. In particular, the user 108 may create / define size-based categories and display detections with different colors, line thicknesses, shadings, and/or the like based on the different categories/scores.

Turning to Fig. 3, a block diagram of point-of-care ultrasound system 100 configured to assist in the real-time medical imaging analysis of a subject 102 is illustrated in accordance with further aspects of the present disclosure. The system 100 can be configured or adapted for the automatic, size-based analysis of ultrasound cineloops in real-time or substantially real-time with the ultrasound imaging, as described herein.

In the example of Fig. 3, the system 100 comprises an electronic device 104 including one or more processors 305, machine-readable memory 310, an input/output interface 315, and/or a networking unit 320, all of which may be interconnected and/or communicate through a system bus 325 containing conductive circuit pathways through which instructions (e.g., machine-readable signals) may travel to effectuate communication, tasks, storage, and the like. As described above, the electronic device 104 may also include a user input device 106, a display 114, and a user interface 116.

The one or more processors 305 may include one or more high-speed data processors adequate to execute the program components described herein and/or perform one or more steps of the methods described herein. The one or more processors 305 may include an application-specific integrated circuit (ASIC), a field-programmable gate array (FPGA), a microprocessor, a multi-core processor, a multithreaded processor, an ultra-low voltage processor, an embedded processor, and/or the like, including combinations thereof. The one or more processors 305 may include multiple processor cores on a single die and/or may be a part of a system on a chip (SoC) in which the processor 305 and other components are formed into a single integrated circuit, or a single package. As a non-exhaustive list, the one or more processors 305 may include one or more of an Intel® Architecture Core™ based processor, such as a Quark™, an Atom™, an i3, an i5, and i7, or an MCU-class processor, an Advanced Micro Devices, Inc. (AMD) processor such as a Ryzen or Epyc based processor, an A5-A10 processor from Apple® Inc., a Snapdragon™ processor from Qualcomm® Technologies, Inc., and/or the like.

The input/output (I/O) interface 315 of the electronic device 104 may include one or more I/O ports that provide a physical connection to one or more devices, such as one or more health monitoring devices. Put another way, the I/O interface 315 may be configured to connect one or more peripheral devices of the system 100 in order to facilitate communication and/or control of between such devices. In particular embodiments, the system 100 includes an ultrasound probe 112 as described above, which may be coupled to the electronic device 104 via the I/O interface 315. In some embodiments, the I/O interface 315 may include one or more serial ports.

The networking unit 320 of the electronic device 104 may include one or more types of networking interfaces that facilitate wireless communication between one or more components of the electronic device 104 and/or between the electronic device 104 and one or more external components. In embodiments, the networking unit 320 may operatively connect the electronic device 104 to a communications network (340), which may include a direct interconnection, the Internet, a local area network ("LAN"), a metropolitan area network ("MAN"), a wide area network ("WAN"), a wired or Ethernet connection, a wireless connection, a cellular network, and similar types of communications networks, including combinations thereof. In some examples, electronic device 104 may communicate with one or more remote data storage repositories 345, including but not limited to remote / cloud-based servers, cloud-based services, and/or wireless devices via the networking unit 320.

The memory 310 of the electronic device 104 can be variously embodied in one or more forms of machine-accessible and machine-readable storage media. In some examples, the memory 310 may include one or more types of memory, including one or more types of transitory and/or non-transitory memory. In particular embodiments, the memory 310 may include a magnetic disk storage device, an optical disk storage device, an array of storage devices, a solid-state memory device, and/or the like, including combinations thereof. The memory 310 may also include one or more other types of memory, such as dynamic random-access memory (DRAM), static random-access memory (SRAM), erasable programmable read-only memory (EPROM), electrically erasable programmable read-only memory (EEPROM), Flash memory, and/or the like.

In particular embodiments, the memory 310 can be configured to store data 330 and machine-readable instructions 335 that, when executed by the one or more processors 305, cause the electronic device 104 to perform one or more steps of the methods and/or processes described herein. Put another way, provided herein is a computer-readable storage medium 310 having stored thereon machine-readable instructions 335 to be executed by one or more processors 305, and one or more processors 305 configured by the machine-readable instructions 335 stored on the computer-readable storage medium 310 to perform one or more of the operations of the methods described herein.

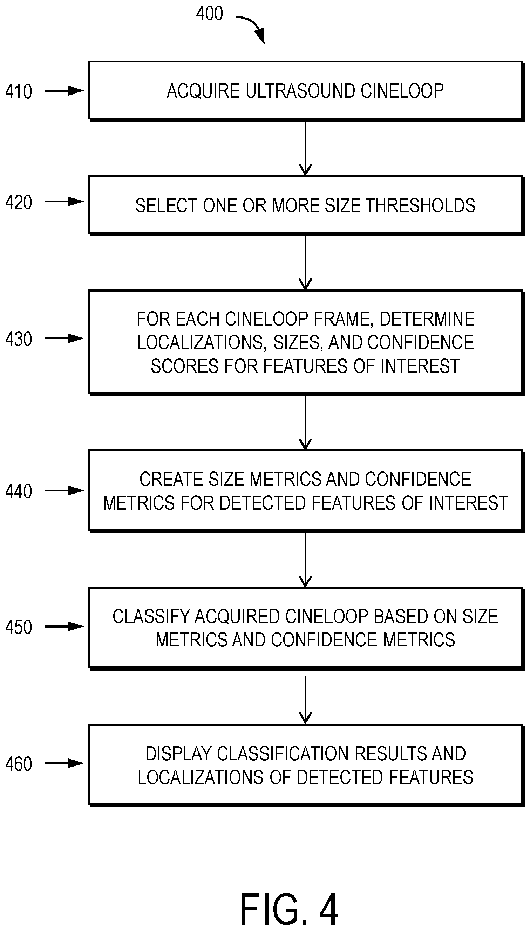

For example, with reference to Fig. 4, a computer-implemented method 400 for automated, size-based analysis of ultrasound cineloops using a point-of-care ultrasound system 100 having an electronic device 104 that is local (i.e., in proximity to) a subject 102 undergoing ultrasound imaging is illustrated according to certain aspects of the present disclosure. As shown, the method 400 includes: in a step 410, acquiring, via an ultrasound probe 112 coupled to the electronic device 104, ultrasound imaging data 110 of the subject 102 in real-time or substantially real-time, wherein the ultrasound imaging data 110 includes an ultrasound cineloop comprising a plurality of ultrasound imaging frames; in a step 420, receiving, via a user input device 106, a user input comprising a user selection of one or more size thresholds; in a step 430, for each cineloop frame, determining localizations, sizes, and/or confidence scores for one or more features-of-interest; in a step 440, creating size-based metrics and confidence metrics for the detected features-of-interest; in a step 450, classify the acquired cineloop based on the size-based metrics and confidence metrics; and in a step 460, display the classification results and localizations of the detected features-of-interest.

In particular embodiments, the step 410 of the method 400 includes receiving, from an ultrasound probe 112 coupled to the electronic device 104, ultrasound imaging data 110 of the subject 102 in real-time or substantially real-time, wherein the ultrasound imaging data 110 includes one or more ultrasound cineloops, each cineloop comprising a plurality of ultrasound imaging frames. As mentioned above, an entire acquired cineloop may include a sequence of 20 to 200 ultrasound imaging frames taken over a short period of time, and a typical LUS exam includes acquiring multiple cineloops (up to 12 or 14 cineloops in a complete, standardized lung exam). Additionally, the LUS exam may include cineloops taken within different zones of the thorax (i.e., the thorax of the subject 102), where there may be anywhere from 2 to 14 zones (or more) covering different regions of the thorax. The ultrasound imaging data 110 may be received following imaging data acquisition by the ultrasound probe 112.

In embodiments, the step 420 of the method 400 includes receiving, via a user input device 106, a user input. In particular embodiments, the user input can include one or more selections (i.e., user selections) of one or more size thresholds. In further embodiments, the user input may include a user selection of at least one size threshold corresponding to at least a first type of feature-of-interest. In some embodiments, the user may select at least one size threshold by accepting a default threshold. That is, for example, the user input may include a selection or confirmation of a default setting (e.g., "Consolidations smaller/larger than 1 cm in height or width", or "Pleural Effusions smaller/larger than 0.5 cm in height", etc.). In embodiments, one or more default settings may be predefined based on one or more clinical contextual parameters associated with the patient 102, such as the patient's age, height, weight, sex / gender, stage of physical development, and/or the like. Accordingly, in particular embodiments, the user input may include a selection of one or more clinical contextual parameters associated with the patient 102. Based on the selection of a clinical contextual parameter or a combination thereof, the electronic device 104 may be configured to apply one or more predefined (i.e., default) size thresholds.

In certain embodiments, the step 420 of receiving a user input can further comprise: generating, via the electronic device 104, a user interface 116 comprising an interactive menu for receiving the user selection of one or more size thresholds, wherein the one or more size thresholds includes at least a feature height threshold, a feature width threshold, and/or a feature area threshold; and displaying, via a display 114 of the electronic device 104, the user interface 116.

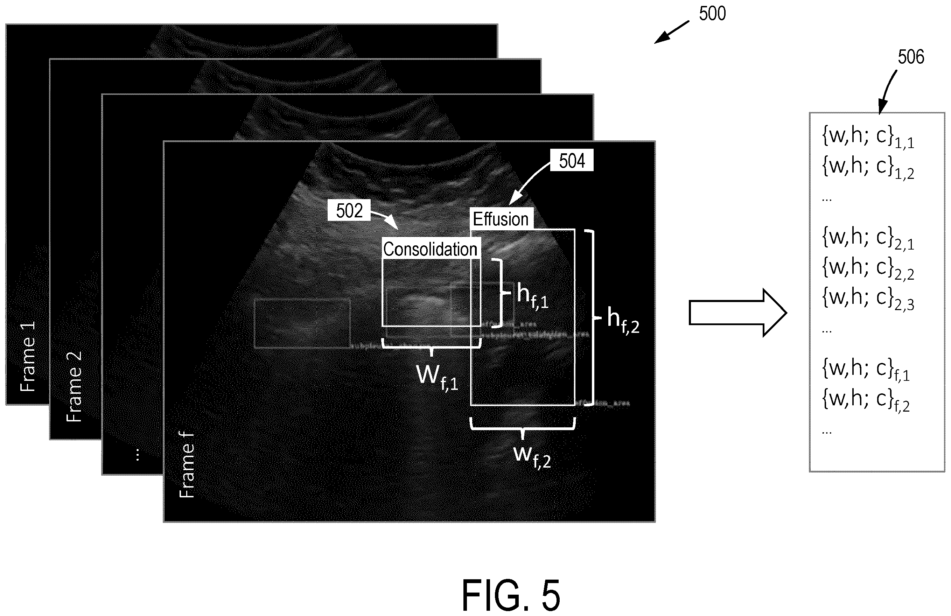

In embodiments, the step 430 of the method 400 includes analyzing the imaging frames of the cineloops to determine localizations, sizes, and confidence scores for one or more features-of-interest. In particular embodiments, the ultrasound cineloop can be analyzed in real-time or substantially real-time relative to the acquisition of the cineloops (ideally within seconds) using one or more detection algorithms and/or trained artificial intelligence models. For example, with reference to Fig. 5, several frames of a cineloop 500 are shown where a consolidation 502 and an effusion 504 are localized across multiple frames of the cineloop 500, and size measurements 506 (including associated confidence levels) are determined for each frame of the cineloop 500.

In some embodiments, the step 440 of the method 400 can include creating size-based metrics / thresholds associated with one or more types of features. Put another way, the thresholds / conditions required applied in the subsequent classification step can be created by the physician-user 108 in order to adapt the sensitivity or specificity of the classification to the clinical context (e.g., the physician can directly enter a threshold value, or choose options such as "adult" vs. "pediatric" which in turn will result in different thresholds being applied). The size categories or size scores may be calculated and displayed on a per-object or per-frame basis, and/or as a single measurement for the entire cineloop. In other embodiments, the classification definitions may be predetermined and only selected based on the user input received.

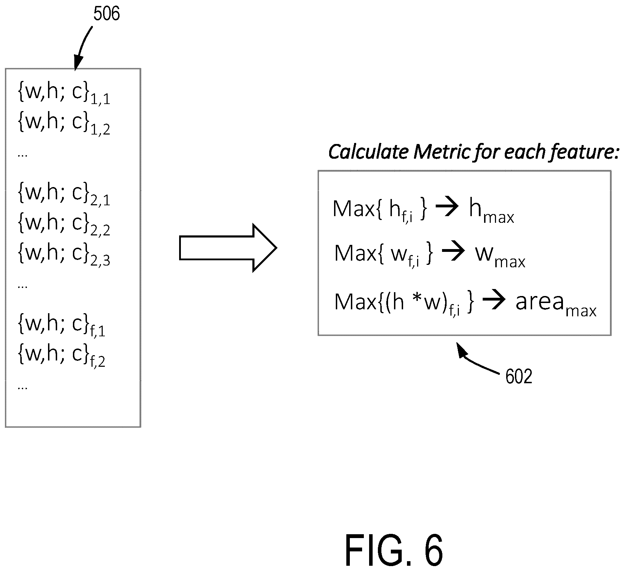

In the step 450, the method 400 then includes classifying the acquired cineloop based on the size measurements and metrics determined. That is, in particular embodiments, the method 400 can include analyzing each of the one or more features-of-interest identified to determine a set of size metrics associated with a corresponding feature-of-interest. For example, as shown in Fig. 6, the measurements 506 determined for each feature-of-interest are used to determine a set 506 of size metrics including one or more statistical parameters for the corresponding feature-of-interest. As described herein, the statistical parameters may be determined based on the set of detection parameters. For example, for each feature-of-interest detected in an ultrasound cineloop, a statistical parameter (e.g., minimum and/or maximum) may be determined for the measurable parameter (e.g., height, width, and/or area) of the feature-of-interest.

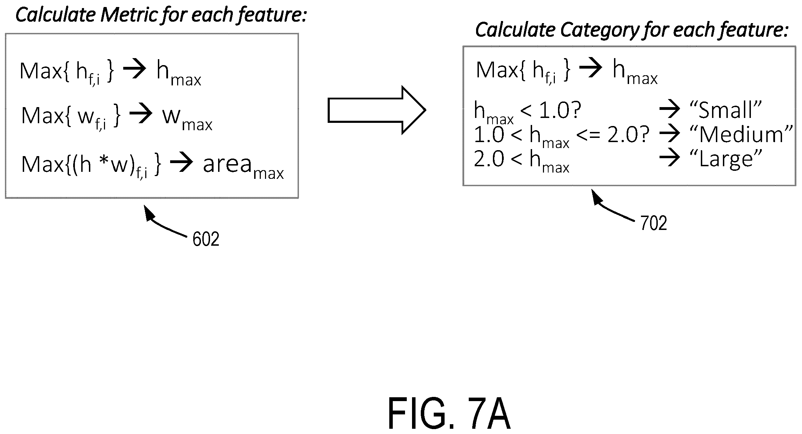

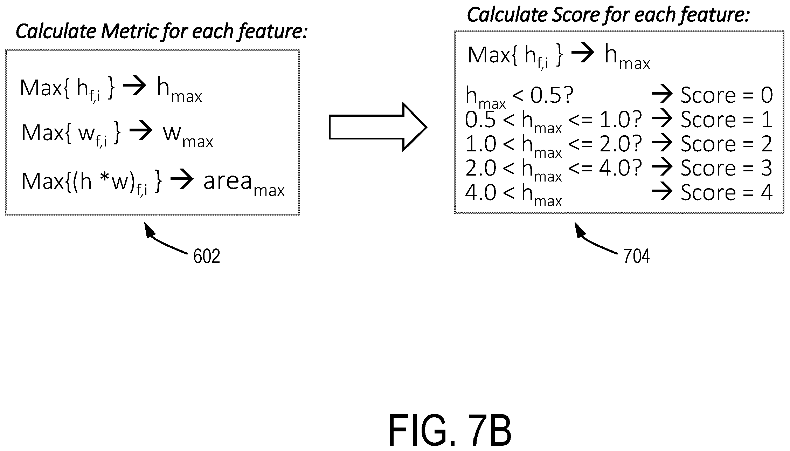

Then, the step 450 may include determining a classification output for the ultrasound cineloop based on the classification definition 702, 704. For example, as shown in Figs. 7A and 7B, a classification definition 702, 704 can be created or predefined based on the user selection of one or more size thresholds, and therefore the classification output (e.g., "small", "medium", "large", etc.) are also based on the user input.

In a step 460, the method 400 then includes, displaying the classification results and localizations of the detected features. Put another way, the step 460 includes outputting the classification output to a user 108 associated with the point-of-care ultrasound system 100. In particular embodiments, the displaying the classification results and localizations of the detected features can include: generating, via the electronic device 104, a user interface 116 comprising (A) one or more ultrasound imaging frames 202 of the ultrasound cineloop 500, (B) a set of annotations 204, 206 indicating the one or more features-of-interest identified, and (C) the classification output 208 for the ultrasound cineloop 500, wherein the classification output includes a feature classification 208 determined based on the user selection of one or more size thresholds and the sets of sizes metrics associated with the one or more features-of-interest; and displaying, via a display 114 of the electronic device 104, the user interface 116.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one." In the claims, any reference signs placed between parentheses shall not be construed as limiting the claim. Measures recited in mutually different dependent claims may advantageously be used in combination.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "having," "containing," "involving," "holding," and the like are to be understood to be open-ended, i.e., to mean including but not limited to.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer-readable storage medium (or media) having computer-readable program instructions thereon for causing a processor to carry out aspects of the present disclosure.

The computer-readable storage medium can be a tangible device that can retain and store instructions for use by an instruction execution device. The computer-readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer-readable program instructions from the network and forwards the computer-readable program instructions for storage in a computer-readable storage medium within the respective computing/processing device.

Computer-readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as C++, or the like, and procedural programming languages, such as the "C" programming language or similar programming languages. The computer-readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer-readable program instructions by utilizing state information of the computer-readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer-readable program instructions.

The computer-readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions can also be stored in a computer-readable storage medium that can direct a computer, a programmable data processing apparatus, and/or other devices to function in a particular manner, such that the computer-readable storage medium having instructions stored therein comprises an article of manufacture comprising instructions which implement aspects of the function/act specified in the flowchart and/or block diagram or blocks.

The computer-readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The computer program product may be software available for download from a server, e.g., via the internet. Alternatively, the computer program product may be a suitable (non-transitory) computer-readable medium on which the instructions are stored, such as an optical storage medium or a solid-state medium, which may or may not be supplied together with or as part of other hardware.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

**Claims**

1. An ultrasound imaging system (100) configured to assist in the real-time medical imaging analysis of a subject (102), the system (100) comprising:
an electronic device (104) comprising:

a user input device (106) configured to receive user input from a user (108); and
one or more processors (305) configured to:

(i) receive ultrasound imaging data (110) in real-time or substantially real-time, wherein the ultrasound imaging data (110) includes an ultrasound cineloop comprising a plurality of ultrasound imaging frames;
(ii) receive a user input via the user input device (106), wherein the user input includes a user selection of one or more size thresholds;
(iii) analyze the ultrasound cineloop in real-time or substantially real-time using one or more trained artificial intelligence models to identify one or more features-of-interest;
(iv) analyze each of the one or more features-of-interest identified to determine a set of size metrics associated with a corresponding feature-of-interest; and
(v) determine a classification output for the ultrasound cineloop based on the user selection of one or more size thresholds and the sets of size metrics associated with the one or more features-of-interest.

2. The ultrasound imaging system (100) of claim 1, further comprising:

an ultrasound probe (112) coupled to the electronic device (104); and
wherein the one or more processors (305) are further configured by the instructions to:
receive ultrasound imaging data (110) from the ultrasound probe.

3. The ultrasound imaging system (100) of claim 1 or 2, wherein the electronic device (104) further comprises:
a display (114), and
wherein the one or more processors (305) are further configured to:
(vi) display, on the display (114), a user interface (116) comprising

(A) one or more ultrasound imaging frames (202) of the ultrasound cineloop,
(B) a set of annotations (204, 206) indicating the one or more features-of-interest identified, and
(C) the classification output (208, 210) for the ultrasound cineloop, wherein the classification output (208, 210) includes a feature classification determined based on the user selection of one or more size thresholds and the sets of size metrics associated with the one or more features-of-interest.

4. The ultrasound imaging system (100) of claim 3, wherein the set of annotations (204, 206) comprises:

(A) one or more bounding boxes (204) identifying a region within at least one ultrasound imaging frame of the ultrasound cineloop, wherein the region contains at least one of the one or more features-of-interest; and
(B) a feature type label (206) associated with at least one of the one or more features-of-interest identified.

5. The ultrasound imaging system (100) of claim 4, wherein the user input received via the user input device (106) includes a user selection of at least one size threshold corresponding to at least a first type of feature-of-interest.

6. The ultrasound imaging system (100) of any one of claims 3 to 5, wherein:

the display (114) is a touch-enabled display,
the user input device (106) includes the touch-enabled display, and
the user input includes touch data received via the touch-enabled display.

7. A computer program product comprising instructions (335) that, when executed by one or more processors (305), cause the one or more processors (305) to:

(i) receive ultrasound imaging data (110) in real-time or substantially real-time, wherein the ultrasound imaging data (110) includes an ultrasound cineloop comprising a plurality of ultrasound imaging frames;
(ii) receive a user input via a user input device (106), wherein the user input includes a user selection of one or more size thresholds;
(iii) analyze the ultrasound cineloop in real-time or substantially real-time using one or more trained artificial intelligence models to identify one or more features-of-interest;
(iv) analyze each of the one or more features-of-interest identified to determine a set of size metrics associated with a corresponding feature-of-interest; and
(v) determine a classification output (208, 210) for the ultrasound cineloop based on the user selection of one or more size thresholds and the sets of size metrics associated with the one or more features-of-interest.

8. The computer program product of claim 7, further comprising instructions that, when executed by the one or more processors (305), cause the one or more processors (305) to:

(vi) generate a user interface (116) comprising:

(A) one or more ultrasound imaging frames (202) of the ultrasound cineloop,
(B) a set of annotations (204, 206) indicating the one or more features-of-interest identified, and
(C) the classification output (208, 210) for the ultrasound cineloop, wherein the classification output (208, 210) includes a feature classification determined based on the user selection of one or more size thresholds and the sets of size metrics associated with the one or more features-of-interest; and

(vii) display, on a display (114) associated with the non-transitory computer-readable storage medium (335), the user interface (116).

**9.** The computer program product of claim 8, wherein the set of annotations (204, 206) comprises:

(A) one or more bounding boxes (204) identifying a region within at least one ultrasound imaging frame (202) of the ultrasound cineloop, wherein the region contains at least one of the one or more features-of-interest; and
(B) a feature type label (206) associated with at least one of the one or more features-of-interest identified.

**10.** The computer program product (310) of claim 7, wherein the user input received via the user input device (106) includes a user selection of at least one size threshold corresponding to at least a first type of feature-of-interest.

**11.** A computer-implemented method (400) for automated, size-based analysis of ultrasound cineloops using an ultrasound imaging system (100) having an electronic device (104) that is local to a subject (102) undergoing ultrasound imaging, the method (400) comprising:

receiving (410), from an ultrasound probe (112) coupled to the electronic device (104), ultrasound imaging data (110) of the subject (102) in real-time or substantially real-time, wherein the ultrasound imaging data (110) includes an ultrasound cineloop comprising a plurality of ultrasound imaging frames;
receiving (420), from a user input device (106), a user input comprising a user selection of one or more size thresholds;
analyzing (430) the ultrasound cineloop in real-time or substantially real-time using one or more trained artificial intelligence models to identify one or more features-of-interest;
analyzing (440) each of the one or more features-of-interest identified to determine a set of size metrics associated with a corresponding feature-of-interest;
determining (450) a classification output (208, 210) for the ultrasound cineloop based on the user selection of one or more size thresholds and the sets of size metrics associated with the one or more features-of-interest; and
outputting (460), via the electronic device (104), the classification output (208, 210) to a user (108) associated with the ultrasound imaging system (100).

**12.** The computer-implemented method (400) of claim 11, further comprising:

generating a user input interface comprising an interactive menu for receiving the user selection of one or more size thresholds, wherein the one or more size thresholds includes at least a feature height threshold, a feature width threshold, and/or a feature area threshold; and
displaying, on a display (114) of the electronic device (104), the user input interface;
wherein the user input device (106) comprises the user input interface.

**13.** The method (400) of claim 11 or 12, further comprising:

generating a graphical user interface (116) comprising

(A) one or more ultrasound imaging frames (202) of the ultrasound cineloop,
(B) a set of annotations (204, 206) indicating the one or more features-of-interest identified, and
(C) the classification output (208, 210) for the ultrasound cineloop, wherein the classification output (208, 210) includes a feature classification determined based on the user selection of one or more size thresholds and the sets of sizes metrics associated with the one or more features-of-interest; and

displaying, on a display (114) of the electronic device (104), the graphical user interface (116);
wherein the classification output (208, 210) is outputted to the user (108) associated with the ultrasound imaging system (100) as part of the graphical user interface (116) displayed on the display (114) of the electronic device (104).

**14.** The method (400) of claim 13, wherein

the set of annotations (204, 206) is automatically generated based on the one or more features-of-interest identified by the one or more artificial intelligence models, and
the set of annotations comprise:

(A) one or more bounding boxes (204) identifying a region within at least one ultrasound imaging frame of the ultrasound cineloop, wherein the region contains at least one of the one or more features-of-interest; and

(B) a feature type label (206) associated with at least one of the one or more features-of-interest identified

15. The method (400) of any one of claims 11 to 14, wherein one or more features-of-interest identified by analyzing the ultrasound cineloop using one or more trained artificial intelligence models are excluded from the classification output (208, 210) based on the user selection of one or more size thresholds and the sets of size metrics associated with the excluded features-of-interest.

FIG. 1

104

114

208

116

Point-of-Care Ultrasound

Consolidation:

Small

Effusion:

Medium

Feature 3:

....

210

Consolidation

Effusion

206

204

Frame 1

Frame 2

Frame f

202

FIG. 2A

FIG. 2B

EP 4 501 245 A1

FIG. 3

400

| 410 → | ACQUIRE ULTRASOUND CINELOOP |
|---|---|

| 420 → | SELECT ONE OR MORE SIZE THRESHOLDS |
|---|---|

| 430 → | FOR EACH CINELOOP FRAME, DETERMINE LOCALIZATIONS, SIZES, AND CONFIDENCE SCORES FOR FEATURES OF INTEREST |
|---|---|

| 440 → | CREATE SIZE METRICS AND CONFIDENCE METRICS FOR DETECTED FEATURES OF INTEREST |
|---|---|

| 450 → | CLASSIFY ACQUIRED CINELOOP BASED ON SIZE METRICS AND CONFIDENCE METRICS |
|---|---|

| 460 → | DISPLAY CLASSIFICATION RESULTS AND LOCALIZATIONS OF DETECTED FEATURES |
|---|---|

# FIG. 4

FIG. 5

EP 4 501 245 A1

506

$\{w, h; c\}_{1,1}$
$\{w, h; c\}_{1,2}$
...

$\{w, h; c\}_{2,1}$
$\{w, h; c\}_{2,2}$
$\{w, h; c\}_{2,3}$
...

$\{w, h; c\}_{f,1}$
$\{w, h; c\}_{f,2}$
...

*Calculate Metric for each feature:*

$\text{Max}\{ h_{f,i} \} \rightarrow h_{max}$

$\text{Max}\{ w_{f,i} \} \rightarrow w_{max}$

$\text{Max}\{(h * w)_{f,i} \} \rightarrow area_{max}$

602

FIG. 6

**Calculate Metric for each feature:**

$$\text{Max}\{ h_{f,i} \} \rightarrow h_{max}$$

$$\text{Max}\{ w_{f,i} \} \rightarrow w_{max}$$

$$\text{Max}\{(h \ast w)_{f,i} \} \rightarrow area_{max}$$

602

**Calculate Category for each feature:**

$$\text{Max}\{ h_{f,i} \} \rightarrow h_{max}$$

$h_{max} < 1.0?$ $\rightarrow$ "Small"
$1.0 < h_{max} <= 2.0?$ $\rightarrow$ "Medium"
$2.0 < h_{max}$ $\rightarrow$ "Large"

702

# FIG. 7A

EP 4 501 245 A1

*Calculate Metric for each feature:*

$$\text{Max}\{\, h_{f,i} \,\} \rightarrow h_{max}$$

$$\text{Max}\{\, w_{f,i} \,\} \rightarrow w_{max}$$

$$\text{Max}\{(h * w)_{f,i}\} \rightarrow area_{max}$$

602

*Calculate Score for each feature:*

$$\text{Max}\{\, h_{f,i} \,\} \rightarrow h_{max}$$

$h_{max} < 0.5?$ → Score = 0
$0.5 < h_{max} <= 1.0?$ → Score = 1
$1.0 < h_{max} <= 2.0?$ → Score = 2
$2.0 < h_{max} <= 4.0?$ → Score = 3
$4.0 < h_{max}$ → Score = 4

704

FIG. 7B

Europäisches
Patentamt
European
Patent Office
Office européen
des brevets

# EUROPEAN SEARCH REPORT

Application Number

EP 23 19 4483

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 109 528 230 A (JINAN INSPUR HI TECH INVEST & DEVELOPMENT CO LTD) 29 March 2019 (2019-03-29) * paragraph [0002] – paragraph [0004] * * paragraph [0022] – paragraph [0024] * ----- | 1-15 | INV. A61B8/08 A61B8/00 |
| Y | US 2016/022238 A1 (PARK JIN MAN [KR]) 28 January 2016 (2016-01-28) * paragraph [0043] * * paragraph [0051] * * paragraph [0059] * * paragraph [0060] * * figure 4 * ----- | 1-15 | |

**TECHNICAL FIELDS SEARCHED (IPC)**

A61B

The present search report has been drawn up for all claims

| Place of search | Date of completion of the search | Examiner |
|---|---|---|
| Munich | 12 February 2024 | Ordavo, Ivan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

..............................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

**EP 23 19 4483**

This annex lists the patent family members relating to the patent documents cited in the above-mentioned European search report.
The members are as contained in the European Patent Office EDP file on
The European Patent Office is in no way liable for these particulars which are merely given for the purpose of information.

**12-02-2024**

| Patent document cited in search report | | | Publication date | Patent family member(s) | | | Publication date |
|---|---|---|---|---|---|---|---|
| CN 109528230 | | A | 29-03-2019 | NONE | | | |
| US 2016022238 | | A1 | 28-01-2016 | KR | 20160012758 | A | 03-02-2016 |
| | | | | US | 2016022238 | A1 | 28-01-2016 |

EPO FORM P0459

For more details about this annex : see Official Journal of the European Patent Office, No. 12/82

**REFERENCES CITED IN THE DESCRIPTION**

**Patent documents cited in the description**

- US 63437206